**Europäisches Patentamt**

(19) **European Patent Office**

**Office européen des brevets**

(11) Publication number: **0 131 768**
**A2**

## (12) EUROPEAN PATENT APPLICATION

(21) Application number: **84106883.6**

(22) Date of filing: **15.06.84**

(51) Int. Cl.⁴: **B 23 K 26/02**

(30) Priority: **17.06.83 US 505255**

(43) Date of publication of application:
**23.01.85 Bulletin 85/4**

(84) Designated Contracting States:
**AT BE CH DE FR GB IT LI LU NL SE**

(71) Applicant: **Coherent, Inc.**
**3210 Porter Drive**
**Palo Alto, Cal. 94303(US)**

(72) Inventor: **Hennings, David Robert**
**315 Escuela, Apt. 150**
**Mountain View, CA 94040(US)**

(72) Inventor: **Fischer, Dennis G.**
**14205 Edgehill Lane**
**Auburn, CA 95603(US)**

(74) Representative: **Reinländer & Bernhardt Patentanwälte**
**Orthstrasse 12**
**D-8000 München 60(DE)**

(54) **A method and an apparatus for focusing an invisible beam of electromagnetic radiation.**

(57) The present invention relates to a method and an apparatus for focusing an invisible beam of coherent radiation at a desired spot. The invisible beam of coherent radiation is combined with a visible beam of coherent radiation to form a combined beam. The combined beam comprises the visible beam and the invisible beam substantially collinear with one another. The combined beam is then expanded and is filtered. The filter passes substantially all of the invisible radiation and only a portion of the visible radiation. The portion of the visible radiation passed forms two indicia which are located off the axis of the combined beam. The filtered beam is then passed through a focusing lens. At the desired spot, when the two indicia of visible beam converged to form a single indicium, then the invisible beam of radiation will also be brought to a focus at that spot.

FIG.—2.

0131768

## Description

## A Method And An Apparatus For Focusing An Invisible Beam Of Electromagnetic Radiation

### Technical Field

The present invention relates to a method and an apparatus for detecting when an invisible beam of electromagnetic radiation is in focus. More particular, the present invention relates to a method and an apparatus for focusing an invisible beam of coherent eletromagnetic radiation.

### Background Of The Invention

A YAG laser is well-known in the art. A YAG laser generates a beam of coherent electromagnetic radiation at approximately 1.06 microns. At that frequency, the light is in the near infrared range and is invisible to the human eye. The YAG laser has found use in many medical applications. One such application is the use of a YAG laser delivered to an eye of a patient for surgical purposes, i.e. precisely cut portions of the eye.

Since the output of a YAG laser is a beam of light which is invisible to the human eye, it becomes necessary to determine when the beam of invisible radiation from the YAG is properly focused at the desired location. One such apparatus and method is disclosed in a device manufactured by Lasag Corporation. However, that device first expands the beam from the YAG laser. A beam of visible radiation produced by a HeNe laser is split into two beams. The two beams from the HeNe laser are then combined with the expanded beam from the YAG laser. The combined beam is then directed at a focusing lens which brings the combined beam to a desired focal point. The invisible beam of coherent radiation from the YAG laser is at a focus when the two beams of visible radiation from the HeNe laser converge into a single spot. This device requires that the two beams of HeNe laser be precisely combined with the beam of invisible radiation from the YAG laser. Therefore, the potential for misalignment is great in such a system.

Summary Of The Invention

In accordance with the present invention, an improved method and apparatus for focusing an invisible beam of electromagnetic radiation is disclosed. In this invention, the invisible radiation is generated. A beam of visible radiation is also generated. A combined beam is formed from the invisible beam and the visible beam. The combined beam has an axis such that the invisible beam and the visible beam are coaxial with the axis of the combined beam. The combined beam is expanded. The expanded beam is then filtered by passing substantially all of the invisible radiation and by passing only a portion of the visible radiation. The portion of the visible beam being passed is such that at least two indicia positioned off the axis of the combined beam are passed. The filter beam is then focused by a focusing means and is moved until the indicia are substantially in registry with one another, whereby the beam of invisible radiation is then in focus.

Brief Description Of The Drawings

Fig. 1 is a schematic view of what is believed to be prior art system.

Fig. 2 is a schematic view of the apparatus of the present invention.

Fig. 3a and 3b are the front and side view, respectively, of a filter used in the present invention.

Fig. 4 is a plot of the optical properties of the filter in the present invention.

Fig. 5 is a plot of the optical properties of the anti-reflective coating in the filter in the present invention.

Detailed Description Of The Drawings

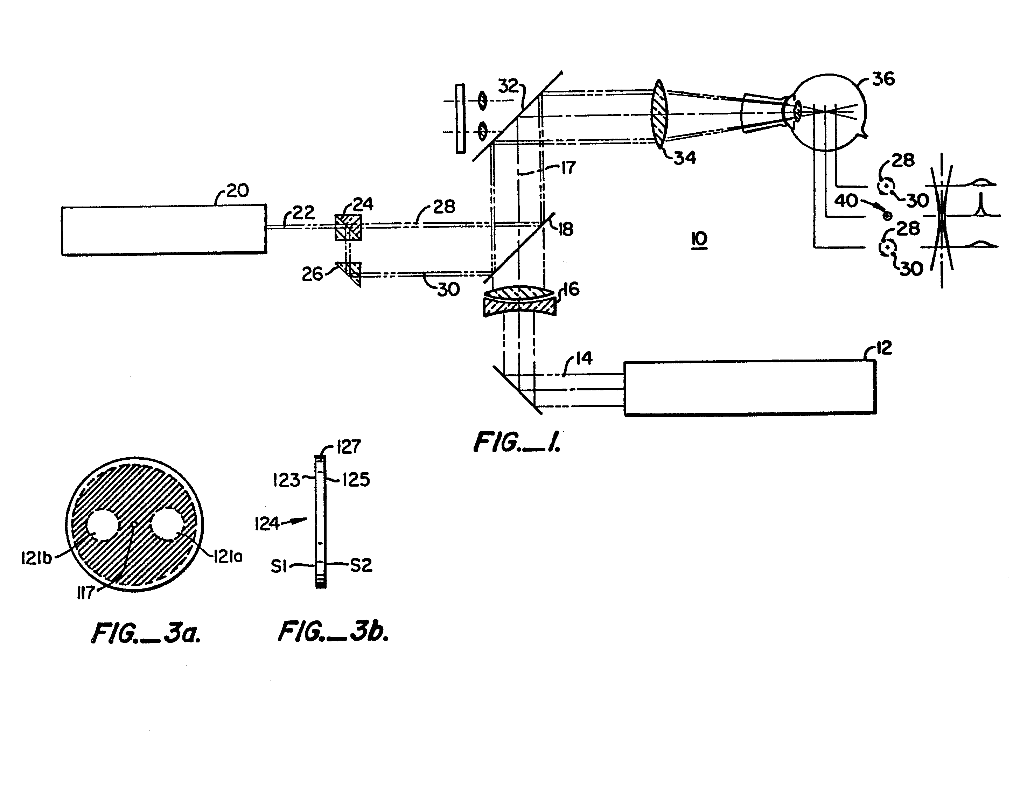

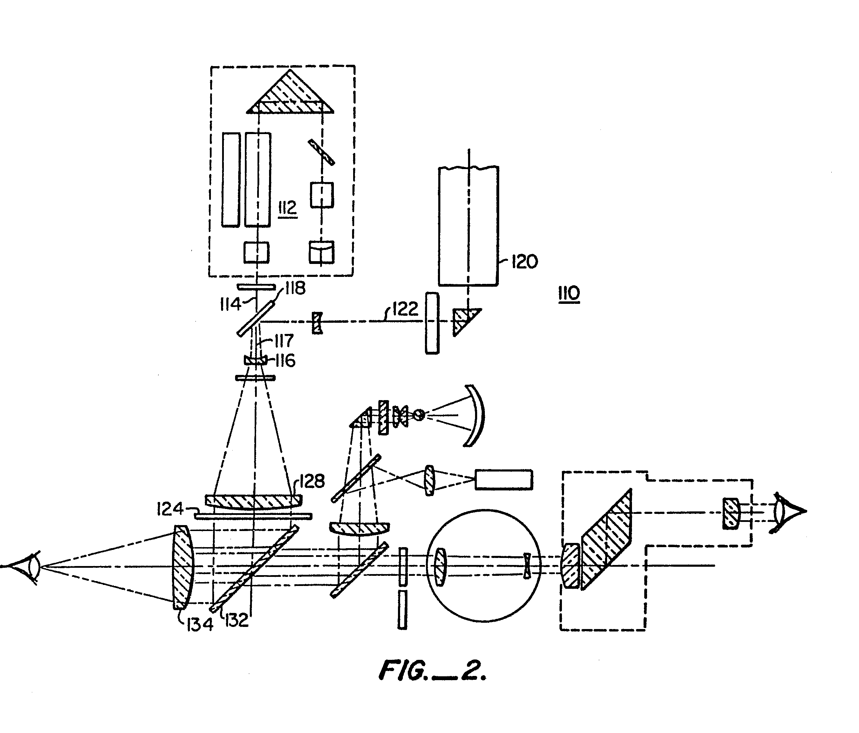

Referring to Fig. 1, there is shown a schematic view of what is believed to the prior art apparatus. In Fig. 1, the apparatus 10 comprises a YAG laser 12 for generating a beam 14 of radiation at approximately 1.06 microns which is invisible to the human eye. The invisible beam 14 is aligned to impinge a beam expander 16. The beam expander 16 expands the invisible beam 14. From the beam expander 16, the expanded beam 14 impinges a partial reflector 18.

A HeNe laser 20 generates a beam 22 of visible radiation. Typically, the HeNe laser generates a visible beam at .6328 microns. At this frequency, the beam 22 of coherent radiation from the HeNe laser 20 is visible to the human eye. The visible beam 22 is aligned to impinge a beam splitting cube 24 which splits the beam into two beams: 28 and 30. The two visible beams 28 and 30 are spaced apart and parallel to one another, and are aligned to impinge the reflector 18. The expanded invisible beam 14 from the YAG laser 12 is combined with the two visible beams 28 and 30 at the reflector 18. The combined beam from the reflector 18 has a central axis 17. The invisible expanded beam 14 is coaxial with the central axis 17 of the combined beam. The two beams 28 and 30 from the HeNe laser 20 are positioned symmetrically off the central axis 17 and near the periphery of the expanded invisible beam 14.

The combined beam, comprising the expanded invisible beam 14 from the YAG laser 12, and the two

The combined beam, comprising the expanded invisible beam 14 from the YAG laser 12, and the two visible beams 28 and 30 from the HeNe laser 20 impinge a mirror 32 and a focusing lens 34. The focusing lens 34 focuses the combined beam at a desired object, such as an eye 36.

In the operation of the apparatus 10, the HeNe laser 20 is first turned on generating the two beams 28 and 30. The objective lens 34 is moved parallel to the axis 17 until the two beams 28 and 30 converge and form a single spot 40 at the desired location. At that point, the YAG laser 12 is turned on, generating the invisible beam 14 of radiation. The invisible beam 14 from the YAG laser 12 will be focused at the same location as the single spot 40 from the HeNe laser 20. If the objective lens 34 is moved or the patient has moved relative to the apparatus 10, the radiation from the HeNe laser 20 at the desired spot will appear as two separate distinct beams: 28 and 30. In such event, the YAG laser 12, with its invisible beam 14, will not be brought to a focus at that spot.

The operation of the apparatus 10 of the prior art assumes that the two beams, 28 and 30, from the HeNe laser 20 are correctly aligned with the reflector 18 and with the invisible beam 14 from the YAG laser 12. If there is any movement of the reflector 18 or the relative position of the HeNe laser beams 28 and 30, the invisible beam 14 from the YAG laser 12 will not be brought to a focus at the location where the single spot 40 from the HeNe laser 20 has converged. This is a source of error.

Referring to Fig. 2, there is shown an apparatus 110 of the present invention. The apparatus 110 comprises a YAG laser 112 for generating a beam 114 of invisible radiation. A HeNe laser 120 generates a beam 122 of visible radiation.

The beam 114 of invisible radiation from the YAG laser 112 is aligned to impinge a partial reflector 118. However, unlike the prior art system 10, the beam 114 is not expanded. The visible beam 122 of radiation from the HeNe laser 120 also impinges the reflector 118. Unlike the prior art system 10, the visible beam 122 is not split into two beams. At the partial reflector 118, the beam 114 of invisible radiation and the beam 122 of visible radiation are combined at the same spot. The combined beam from the reflector 118 has an axis 117. The beams 114 and 122 are substantially coaxial with the axis 117 of the combined beam. The combined beam from the reflector 118 is passed through a diverging lens 116 which expands the combined beam. The expanded beam is then passed through a collimating lens 128 which collimates the expanded beam. The expanded, combined beam is then passed through a filter 124, the operation of which will be discussed in greater detail hereinafter. From the filter 124, the filtered, combined, expanded beam is then passed through an objective lens 134 and is brought to a focus at a spot.

Referring to Fig. 3a and Fig. 3b, there is a shown a front and side view, respectively, of the filter 124. The filter 124 comprises a glass window 127 having a first surface 123 and a second surface 125. The collimated beam impinges the first surface 123, passes through the window 127, and exits on the second surface 125. On the first surface 123, the window 127 is coated with a first material in all areas except in two substantially, circularly shaped portions: 121a and 121b, located off the axis 117 of the collimated beam. The material coated on the first surface 123 is of a dielectric material and has substantially greater than 99% reflectance at .632 microns and has greater than 98% transmittance at 1.06 microns. In other words, in from the filter 124. At the same time, the dielectric material will pass the invisible radiation from the YAG laser 112. In the two areas, 121a and 121b, on the first side 123, both the beam 114 from the YAG laser 112 surface the beam 122 from the HeNe 120 will be passed into the window 127. The window 127 passes both of these beams through. On the second surface 125, the window 127 is coated with an anti-reflectance material which prevents the reflection of the radiation impinging thereon. This is to cut down reflection which creates noise and the like. The material on the second surface 125 has greater then 99% transmittance at 1.06 microns and .632 microns. The material is also of a dielectric material.

The material on the first surface 123 comprises a dichroic coating. The material is a combination of layers of material deposited on the window 127 (designated as G), by electron beam vapor deposition. For the material on the first surface 123 the layers are:

```
1.36L
.633H
.633L
.633H
.633L
.633H
.633L
.633H
.633L
.633H
.633L
.633H
.633L
.633H
.633L
.368H
G
```

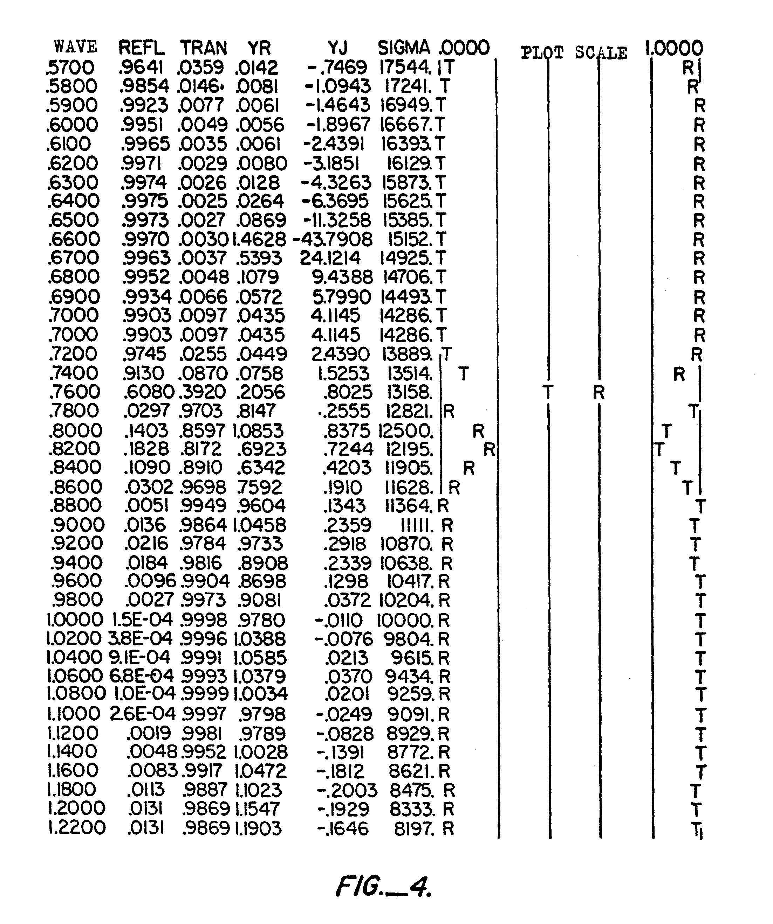

where H is $TiO_2$, L is $SiO_2$ and the numbers are in optical thickness. The optical property of First surface 123 is shown in Figure 4.

The material on the second surface 125 comprises an anti-reflection coating. Similar to the material on the first surface 123, the material on the second surface is a combination of layers of material deposited on the window 127 (designated as G), by electron beam vapor deposition. For the material on the second surface 125, the layers are:

$$.79L$$
$$1.58H$$
$$.79M$$
$$.79L$$
$$G$$

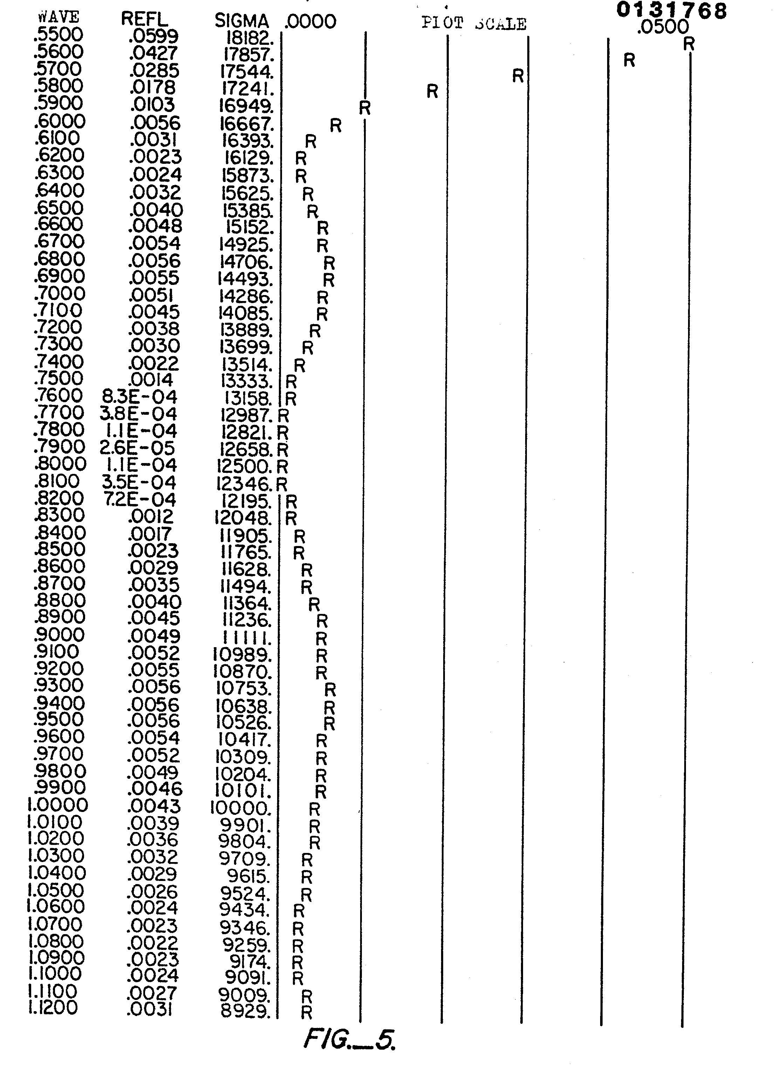

where L is $SiO_2$, M is $Al_2O_3$, H is $HfO_2$, and the numbers are in optical thickness. The optical property of the second surface is shown in Figure 5.

Therefore, from the filter 124, the filtered, combined beam is of an invisible beam 114 from the YAG laser 112 having an axis 117 and with two visible beams 122 positioned off the central axis 117, from the HeNe laser 120.

The two visible spots or beams from the filter 124 serves as indicia. The focusing lens 128 operates similarly to the focusing lens 34 of the prior art.

In the operation of the apparatus 110 of the present invention, similar to the operation of the apparatus 10 of the prior art, when the two indicia or spots of visible radiation 121a and 121b converge to form a single spot, then the invisible beam 114 from the YAG laser 112 will also be focused at that spot.

The only alignment that is required by the apparatus 110 of the present invention is that the visible beam 122 must be combined with the invisible beam 114 at the reflector 118. This can be accomplished in a number of ways. One manner of accomplishing this is by replacing the reflector 118 with a material which renders the invisible beam 114 visible, e.g., photographic paper or thermal paper.

When the invisible beam 114 impinges thereon and produces a spot which is rendered visible, the visible beam 122 is then aligned to impinge at that spot. The paper is then replaced with the reflector 118.

Then, too, since the invisible beam 114 is in the near-infrared region, a radiation converting device, which is well-known in the art, can be placed along the path of a combined beam, i.e., after the reflector 118. When the radiation converting device detects the presence of the invisible beam 114 and of the visible beam 122, the two beams will have been aligned and combined at the reflector 118.

With the method and apparatus of the present invention, the visible and invisible beams are combined prior to their expansion. This greatly facilitates alignment, is more compact and is simpler to use and provides a sharper indication of when the invisible beam 114 is in focus or is out of focus.

Claims:

1. A method for detecting when an invisible beam of electromagnetic radiation is in focus at a desired spot, comprising the steps of:

generating said beam of invisible radiation;

generating a beam of visible radiation;

forming a combined beam from said visible and invisible beams; said combined beam having an axis and being said invisible and visible beams substantially coaxial with said axis;

expanding said combined beam;

filtering said expanded combined beam by passing substantially all of the invisible beam and by passing only a portion of said visible beam; said portion passed being at least two indicia positioned off said axis; and

focusing said filtered beam by moving a focusing means until the indicia are substantially in registry with one another at said desired spot,

whereby said invisible beam is in focus at said spot.

2. The method of Claim 1, wherein said forming step further comprising:

impinging said invisible beam at a location;

rendering said location visible; and

impinging said visible beam at said location.

3.    An apparatus for focusing an invisible beam of electro-magnetic radiation at a desired spot, comprising:

means for generating said beam of invisible radiation;

means for generating a beam of visible radiation;

means for forming a combined beam from said visible and invisible beams; said combined beam having an axis and being said invisible and visible beams substantially coaxial with said axis;

means for expanding said combined beam;

means for filtering said expanded combined beam by passing substantially all of the invisible beam and by passing only a portion of said visible beam; said portion passed being at least two indicia positioned off said axis; and

means for focusing said filtered mean;

whereby said invisible beam being in focus at said spot when said indicia are substantially in registry with one another at said spot.

4.    The apparatus of Claim 3, wherein said beam of invisible radiation is a beam of coherent radiation.

5.    The apparatus of Claim 4, wherein said means for generating said invisible beam is a YAG laser.

6.    The apparatus of Claim 3, wherein said beam of visible radiation is a beam of coherent radiation.

7. The apparatus of Claim 6, wherein said means for generating said visible beam is a HeNe laser.

8. The apparatus of Claim 3, wherein said expanding means comprises a diverging lens.

9. The apparatus of Claim 3, wherein said filter means comprises:

an aperture window having a first face and a second face; said combined beam aligned to impinge said first face to pass through said window and to exit therefrom through said second face; and

a first coating on the surface of said first face except in said portion; said coating being of a material for passing said invisible beam and for reflecting said visible beam.

10. The apparatus of Claim 9 further comprising:

a second coating on the surface of said second face;

said second coating being of a material for passing said visible and invisible beams and for preventing reflection of radiation incident thereon.

0131768

FIG.—1.

FIG.—3a.

FIG.—3b.

FIG._2.

110

120

122

112

114

118

117

116

128

124

132

134

| WAVE | REFL | TRAN | YR | YJ | SIGMA | .0000 | PLOT SCALE | 1.0000 |
|---|---|---|---|---|---|---|---|---|
| .5700 | .9641 | .0359 | .0142 | -.7469 | 17544. | T | | R |
| .5800 | .9854 | .0146 | .0081 | -1.0943 | 17241. | T | | R |
| .5900 | .9923 | .0077 | .0061 | -1.4643 | 16949. | T | | R |
| .6000 | .9951 | .0049 | .0056 | -1.8967 | 16667. | T | | R |
| .6100 | .9965 | .0035 | .0061 | -2.4391 | 16393. | T | | R |
| .6200 | .9971 | .0029 | .0080 | -3.1851 | 16129. | T | | R |
| .6300 | .9974 | .0026 | .0128 | -4.3263 | 15873. | T | | R |
| .6400 | .9975 | .0025 | .0264 | -6.3695 | 15625. | T | | R |
| .6500 | .9973 | .0027 | .0869 | -11.3258 | 15385. | T | | R |
| .6600 | .9970 | .0030 | 1.4628 | -43.7908 | 15152. | T | | R |
| .6700 | .9963 | .0037 | .5393 | 24.1214 | 14925. | T | | R |
| .6800 | .9952 | .0048 | .1079 | 9.4388 | 14706. | T | | R |
| .6900 | .9934 | .0066 | .0572 | 5.7990 | 14493. | T | | R |
| .7000 | .9903 | .0097 | .0435 | 4.1145 | 14286. | T | | R |
| .7000 | .9903 | .0097 | .0435 | 4.1145 | 14286. | T | | R |
| .7200 | .9745 | .0255 | .0449 | 2.4390 | 13889. | T | | R |
| .7400 | .9130 | .0870 | .0758 | 1.5253 | 13514. | T | | R |
| .7600 | .6080 | .3920 | .2056 | .8025 | 13158. | T | R | |
| .7800 | .0297 | .9703 | .8147 | -.2555 | 12821. | R | | T |
| .8000 | .1403 | .8597 | 1.0853 | .8375 | 12500. | R | T | |
| .8200 | .1828 | .8172 | .6923 | .7244 | 12195. | R | T | |
| .8400 | .1090 | .8910 | .6342 | .4203 | 11905. | R | | T |
| .8600 | .0302 | .9698 | .7592 | .1910 | 11628. | R | | T |
| .8800 | .0051 | .9949 | .9604 | .1343 | 11364. | R | | T |
| .9000 | .0136 | .9864 | 1.0458 | .2359 | 11111. | R | | T |
| .9200 | .0216 | .9784 | .9733 | .2918 | 10870. | R | | T |
| .9400 | .0184 | .9816 | .8908 | .2339 | 10638. | R | | T |
| .9600 | .0096 | .9904 | .8698 | .1298 | 10417. | R | | T |
| .9800 | .0027 | .9973 | .9081 | .0372 | 10204. | R | | T |
| 1.0000 | 1.5E-04 | .9998 | .9780 | -.0110 | 10000. | R | | T |
| 1.0200 | 3.8E-04 | .9996 | 1.0388 | -.0076 | 9804. | R | | T |
| 1.0400 | 9.1E-04 | .9991 | 1.0585 | .0213 | 9615. | R | | T |
| 1.0600 | 6.8E-04 | .9993 | 1.0379 | .0370 | 9434. | R | | T |
| 1.0800 | 1.0E-04 | .9999 | 1.0034 | .0201 | 9259. | R | | T |
| 1.1000 | 2.6E-04 | .9997 | .9798 | -.0249 | 9091. | R | | T |
| 1.1200 | .0019 | .9981 | .9789 | -.0828 | 8929. | R | | T |
| 1.1400 | .0048 | .9952 | 1.0028 | -.1391 | 8772. | R | | T |
| 1.1600 | .0083 | .9917 | 1.0472 | -.1812 | 8621. | R | | T |
| 1.1800 | .0113 | .9887 | 1.1023 | -.2003 | 8475. | R | | T |
| 1.2000 | .0131 | .9869 | 1.1547 | -.1929 | 8333. | R | | T |
| 1.2200 | .0131 | .9869 | 1.1903 | -.1646 | 8197. | R | | T |

FIG._4.

0131768

| WAVE | REFL | SIGMA |
|---|---|---|
| .5500 | .0599 | 18182. |
| .5600 | .0427 | 17857. |
| .5700 | .0285 | 17544. |
| .5800 | .0178 | 17241. |
| .5900 | .0103 | 16949. |
| .6000 | .0056 | 16667. |
| .6100 | .0031 | 16393. |
| .6200 | .0023 | 16129. |
| .6300 | .0024 | 15873. |
| .6400 | .0032 | 15625. |
| .6500 | .0040 | 15385. |
| .6600 | .0048 | 15152. |
| .6700 | .0054 | 14925. |
| .6800 | .0056 | 14706. |
| .6900 | .0055 | 14493. |
| .7000 | .0051 | 14286. |
| .7100 | .0045 | 14085. |
| .7200 | .0038 | 13889. |
| .7300 | .0030 | 13699. |
| .7400 | .0022 | 13514. |
| .7500 | .0014 | 13333. |
| .7600 | 8.3E-04 | 13158. |
| .7700 | 3.8E-04 | 12987. |
| .7800 | 1.1E-04 | 12821. |
| .7900 | 2.6E-05 | 12658. |
| .8000 | 1.1E-04 | 12500. |
| .8100 | 3.5E-04 | 12346. |
| .8200 | 7.2E-04 | 12195. |
| .8300 | .0012 | 12048. |
| .8400 | .0017 | 11905. |
| .8500 | .0023 | 11765. |
| .8600 | .0029 | 11628. |
| .8700 | .0035 | 11494. |
| .8800 | .0040 | 11364. |
| .8900 | .0045 | 11236. |
| .9000 | .0049 | 11111. |
| .9100 | .0052 | 10989. |
| .9200 | .0055 | 10870. |
| .9300 | .0056 | 10753. |
| .9400 | .0056 | 10638. |
| .9500 | .0056 | 10526. |
| .9600 | .0054 | 10417. |
| .9700 | .0052 | 10309. |
| .9800 | .0049 | 10204. |
| .9900 | .0046 | 10101. |
| 1.0000 | .0043 | 10000. |
| 1.0100 | .0039 | 9901. |
| 1.0200 | .0036 | 9804. |
| 1.0300 | .0032 | 9709. |
| 1.0400 | .0029 | 9615. |
| 1.0500 | .0026 | 9524. |
| 1.0600 | .0024 | 9434. |
| 1.0700 | .0023 | 9346. |
| 1.0800 | .0022 | 9259. |
| 1.0900 | .0023 | 9174. |
| 1.1000 | .0024 | 9091. |
| 1.1100 | .0027 | 9009. |
| 1.1200 | .0031 | 8929. |

.0000                    PLOT SCALE                    .0500

FIG._5.